(19) **Europäisches Patentamt**
**European Patent Office**
**Office européen des brevets**

(11) Publication number: **0 417 075 B1**

# EUROPEAN PATENT SPECIFICATION

(45) Date of publication of patent specification: **09.06.93** (51) Int. Cl.⁵: **A61K 7/06**

(21) Application number: **88902308.1**

(22) Date of filing: **18.02.88**

(86) International application number:
**PCT/US88/00403**

(87) International publication number:
**WO 89/07436 (24.08.89 89/20)**

(54) **MINOXIDIL GEL.**

(43) Date of publication of application:
**20.03.91 Bulletin 91/12**

(45) Publication of the grant of the patent:
**09.06.93 Bulletin 93/23**

(84) Designated Contracting States:
**AT BE CH DE FR GB IT LI LU NL SE**

(56) References cited:
**EP-A- 0 104 037**
**EP-A- 0 188 793**
**FR-A- 2 590 897**
**FR-A- 2 602 424**
**GB-A- 2 023 000**

(73) Proprietor: **THE UPJOHN COMPANY**
**301 Henrietta Street**
**Kalamazoo, Michigan 49001(US)**

(72) Inventor: **PENA, Lorraine, E.**
**1804 Cambridge Drive**
**Kalamazoo, MI 49001(US)**

(74) Representative: **Perry, Robert Edward et al**
**GILL JENNINGS & EVERY, Broadgate House,**
**7 Eldon Street**
**London EC2M 7LH (GB)**

## Description

Field of the Invention

The present invention relates to a topical gel for the administration of minoxidil.

Background of the Invention

The chemical name for minoxidil is 2,4-diamino-6-piperidinylpyrimidine-3-oxide. It is the active ingredient of Loniten® tablets which are marketed by The Upjohn Company for hypertension; see Physician's Desk Reference, 38th Edition, page 2033 (1984). The preparation and antihypertensive use of this compound are described in US-A-3461461.

Topical pharmaceutical compositions of minoxidil are disclosed in US-A-4139619. The topical compositions comprise minoxidil and a topical pharmaceutical carrier selected from ointments, lotions, pastes, jellies, sprays and aerosols.

Pharmacies in the U.S. have made and sold gels of topical minoxidil containing carbomer, propylene glycol and rubbing alcohol. Similarly, the pharmacists' column "Compounder's Corner", printed in, e.g. "The Missouri Pharmacist", page 35 (January 1987), suggests the preparation of a minoxidil gel containing carbopol, ethanol and water.

Attempts to form pharmaceutically-elegant carbomer gels of minoxidil by conventional means are hampered by three processing difficulties:

(1) the poor solubility of minoxidil;

(2) difficulties in obtaining effective and efficient carbomer dispersion and maintenance of polymer solution; and

(3) precipitation of a drug-carbomer complex.

Conventional carbomer gel formulations involve the sequential mixing of a solvent, a polymer and a neutralizing agent. The drug is added before the neutralising agent is added. This sequence is generally preferred in the preparation of gels.

However, minoxidil cannot be formulated into a carbomer gel by such conventional means. Addition of the components in the conventional manner leads to the formation of a white precipitate which is believed to be a minoxidil-carbomer complex. Isolation and analysis of the precipitate indicate the presence of both carbomer and minoxidil.

FR-A-2602424 discloses a thickened aqueous composition comprising minoxidil, carbomer and less than 20% of a solvent which may be a $C_{1-4}$ alcohol, alkylene glycol, alkylene glycol alkyl ether or dialkylene glycol alkyl ether, and an amine.

EP-A-0188793 discloses an aqueous minoxidil composition and additives selected from carbomer, propylene glycol and ethanol.

GB-A-2023000 discloses an aqueous ointment comprising indomethacine, a glycol, alcohol, carbomer and amine.

Summary of the Invention

A novel composition according to the present invention is a pharmaceutically-acceptable single-phase minoxidil gel, i.e. a minoxidil formulation that is pharmaceutically-elegant. It comprises the following components:

| Component | Amount (%w/w) |
|---|---|
| (a) water | q.s. 100 |
| (b) carbomer | 0.25-1.5 |
| (c) minoxidil | 0.001-3 |
| (d) pharmaceutically-acceptable glycol | 0.01-30 |
| (e) ethanol or isopropanol | 20-40 |
| (f) a water and alcohol-soluble amine | 0.25-1.5 |

wherein the ratio of minoxidil to the glycol is sufficient for a saturated solution of minoxidil.

Surprisingly, and unexpectedly, the three processing difficulties described above can be overcome, and a composition of the invention may be prepared, by a process which comprises a first step of adding 10 to 27.1% w/w of the alcohol and 0 to 0.4% w/w of the amine to a mixture of the water and the carbomer; and a subsequent second step of adding to the mixture of the first step, until a uniform gel is obtained, a mixture of the minoxidil, the glycol, 12.9 to 30% w/w of the alcohol, and at least 0.25% w/w of the amine; the percentages being with respect to the total composition.

Gels are semi-solid systems consisting of either suspensions made up of small inorganic particles or large organic molecules interpenetrated by a liquid.

Single-phase gels, as the term is used herein, consist of organic macromolecules uniformly distributed throughout a liquid in such a manner that no apparent boundaries exist between the dispersed macromolecules and the liquid. Single-phase gels may be made from synthetic macromolecules (e.g. carbomer) or from natural gums (e.g. Tragacanth).

The novel gel is a single-phase gel made from a class of synthetic macromolecules called carbomers. A carbomer is a synthetic, high molecular weight, cross-linked polymer of acrylic acid. Preferred for use in the instant invention is Carbopol® 934P, a pharmaceutical-grade commercial product sold by B.F. Goodrich Company. Carbopol® 934P has an approximate molecular weight of $3 \times 10^6$. Carbomers are well known to those of ordinary skill in the pharmaceutical art.

By "pharmaceutically-acceptable glycol" is meant a glycol which is non-toxic, and does not irritate the skin at the concentrations of this invention. Suitable glycols include propylene glycol, 1,3-butylene glycol, propylene glycol 200 (PEG 200), polyethylene glycol 400 (PEG 400) hexylene glycol, and dipropylene glycol. Propylene glycol is preferred.

The process used to prepare the gel of the instant invention proceeds as follows:

Three components are prepared separately and are mixed as described below. Part I contains carbomer (described in National Formulary (NF)XV at p. 1216 (1980 ed) and commercially available as Carbopol®) and water (preferably purified). This part may be prepared directly from carbomer and water or from a previously prepared dispersion. A second part is prepared containing minoxidil from greater than 0 to 5% on a weight-to-weight basis; propylene glycol and/or 1,3-butylene glycol (1,3-butane diol), and/or other suitable glycol in an amount approximately 10 times that of the minoxidil for propylene glycol or an equivalent saturated amount for other glycols; an alcohol (ethanol or isopropanol) in the range of 12.9 to 30% and an amine which is both water and alcohol soluble, such as diisopropanolamine (DIPA), triisopropanolamine, trolamine (triethanolamine), monoethanolamine or a polyamine such as Quadrol. DIPA, ethanol, and propylene glycol are preferred. The amine should be present in the mixture in the range from about 0.25 to about 1.5%. A third part is prepared containing the above-described alcohol in the range of 27.1 to 10%. A small amount of the amine may also be present in this third part, i.e., up to 0.4% on a weight-to-weight basis. However, it is preferred that no amine be present in Part III. All percents are expressed on a weight-to-weight basis.

Part III is then mixed with Part I by conventional means. After a uniform mixture is obtained, Part II is then added. A planetary mixing action under vacuum is preferred.

Surprisingly and unexpectedly it has been found that this sequence of addition produces a pharmaceutically elegant gel which is not obtainable by conventional means. If the components are added in the conventional manner a white precipitate forms. The key element is the mixture of the amine with the minoxidil in Part II. The process precipitation problem is thus avoided. The preparation of Parts I and III provide for a processible carbomer dispersion and helps to maintain the polymer solution, thereby insuring acceptable gel viscosity and clarity.

In order to produce and maintain drug solubility in the preparation of Part II, the ratio of drug to the propylene glycol must be on the order of approximately 1 to 10 or an equivalent saturated amount for other glycols. A minimum amount of alcohol, determined by total drug concentration, is also required. If the minoxidil is not added simultaneously with the diisopropanolamine (DIPA) or similar amine to the carbomer dispersion, a stringy white precipitate forms. Thus, the addition of the DIPA or similar amine to Part II effectively "masks" the minoxidil and carbopol in such a way that these otherwise incompatable ingredients can be combined without difficulty.

A gel prepared using this process rather than conventional means avoids the problem of poor minoxidil solubility, allows the combination of otherwise incompatible ingredients, and facilitates carbomer dispersion manufacture.

A maximum concentration of alcohol of approximately 40% is preferred for satisfactory gels. This amount of alcohol is preferred to maintain fluidity of the Part I dispersion, since at higher minoxidil concentrations which necessitate a higher glycol content, further reductions in the fluid volume of Part I would result in a non-uniform, "doughy" mass during processing. An alternate reason for limiting the formulation alcohol to 40% is that the gel viscosity is reduced and hazing is increased in response to the carbomer's reaction to an unfavorable, potentially unstable, solvent environment.

Suitable colorants, perfumes, or similar pharmaceutical excipients may be added to the gel to obtain pharmaceutical elegance.

Surprisingly and unexpectedly it has been found that the formulation parameters described herein produce a gel with clarity and acceptable viscosity.

Minoxidil is well known and may be prepared by known means, e.g., as disclosed in U.S. Patent 3,461,461, which is expressly incorporated by reference herein. The minoxidil gels of the instant invention may be used as described in U.S. Patent 4,139,619, which is also expressly incorporated by reference herein.

DESCRIPTION OF THE PREFERRED EMBODIMENTS

The present invention is seen more fully by the Examples given below.

Example 1 Preparation of minoxidil gels.

Pharmaceutically elegant 1, 2, and 3% minoxidil gels are prepared by mixing the below-described 3 part mixtures:

A. Topical minoxidil gel 1%

| Part I | % w/w |
|---|---|
| Purified water USP | q.s. 100 |
| Carbopol® 934P | 0.45 |

| Part II | |
|---|---|
| minoxidil | 1.0 |
| propylene glycol USP | 10 |
| alcohol USP | 13 |
| diisopropanolamine NF | 0.45 |

| Part III | |
|---|---|
| alcohol USP | 27 |

B. Topical minoxidil gel 2%.

| Part I | % w/w |
|---|---|
| purified water USP | q.s. 100 |
| carbopol 934P | 0.5 |

| Part II | |
|---|---|
| propylene glycol USP | 20 |
| alcohol USP | 13 |
| minoxidil | 2 |
| diisopropanolamine NF | 0.5 |

| Part III | |
|---|---|
| alcohol USP | 27 |

C. Topical minoxidil gel 3%.

| Part I | % w/w |
|---|---|
| purified water USP | q.s. 100 |
| carbopol 934P | 0.5 |

| Part II | |
|---|---|
| minoxidil | 3.0 |
| propylene glycol U.S.P. | 30 |
| alcohol USP | 13 |
| diisopropanolamine NF | 0.5 |

| Part III | |
|---|---|
| alcohol USP | 27 |

In each of the above cases, the component parts are prepared separately. Part III is then mixed with Part I. When a uniform mixture is obtained, Part II is then added using planetary mixing under vacuum until a uniform gel is obtained.

Example 2 Topical minoxidil light gel 2%.

82.5 kg of minoxidil gel were prepared as described above using the following quantities in each part:

| % w/w | Part I | Amount |
|---|---|---|
| 0.5% | Carbopol® 934P | 412 g, 500 mg |
|  | Purified Water USP | 30 kg, 937 g, 500 mg |
|  | **Part II** | |
| 20% | Propylene Glycol USP | 16 kg, 500 g |
| (2%) | Minoxidil Milled | (1 kg, 650 g)* |
| 13% | Alcohol USP | 10 kg, 725 g |
| 0.5% | Diisopropanolamine NF | 412 g, 500 mg |
|  | Alcohol USP q.s. ad if necessary to account for evaporation | 29 kg, 287 g, 500 mg |
|  | **Part III** | |
| 27% | Alcohol USP | 22 kg, 275 g |
|  | Alcohol USP q.s. ad | 82 kg, 500 g |

\* Calculated by assay.

Preparation 1 Aqeuous Carbomer Dispersion

A 1.604% Carbomer dispersion was prepared by mixing the following ingredients:

|  | Amount |
|---|---|
| Purified water, USP | 73 kg |
| Carbopol® 934P | 1 kg, 203 gm |
| Purified water, USP q.s. ad | 75 kg |

Mixing was performed in a Nauta mixer under vacuum. An opaque, smooth dispersion resulted.

Example 3 Minoxidil Gel 2%

A 2% minoxidil gel was prepared by mixing the following 3 parts as described below:

| % w/w | Part I | Kg. | Gm. |
|---|---|---|---|
|  | Purified water USP | 6 | 320 |
| 1.604 | Carbopol dispersion (Prep. 1) | 31 | 170 |
|  | **Part II** | | |
| 20 | Propylene glycol USP | 20 | - |

| | | | |
|---|---|---|---|
| 13 | Alcohol USP | 13 | - |
| 2 | Minoxidil milled Assay 99.4% | 2 | 012 |
| 0.5 | DIPA | - | 500 |
| | **Part III** | | |
| 27 | Alcohol USP | 27 | - |
| | Alcohol USP qs ad | 100 | - |

Parts I and III were mixed in a Nauta mixer under vacuum. When a uniform mixture was obtained, the Part II component was added, and mixing was continued under vacuum until a uniform gel was obtained. The product had a pH of 8.04, and a viscosity of 7980 mPa•s (centipoise) at a shear rate of 7.61 $s^{-1}$.

**Claims**

1. A pharmaceutically-acceptable single-phase minoxidil gel composition comprising the following components:

| Component | Amount (% w/w) |
|---|---|
| (a) water | q.s. 100 |
| (b) carbomer | 0.25-1.5 |
| (c) minoxidil | 0.001-3 |
| (d) pharmaceutically-acceptable glycol | 0.01-30 |
| (e) ethanol or isopropanol | 20-40 |
| (f) a water and alcohol-soluble amine | 0.25-1.5 |

wherein the ratio of minoxidil to the glycol is sufficient for a saturated solution of minoxidil.

2. A gel of claim 1, wherein the amine is diisopropanolamine (DIPA), the alcohol is ethanol, the water is purified, and the glycol is propylene glycol.

3. A gel of claim 2, wherein the amount of minoxidil is 1.0%, the amount of carbomer is 0.45%, the amount of propylene glycol is 10%, the amount of ethanol is 40%, and the amount of DIPA is 0.45%.

4. A gel of claim 2, wherein the amount of minoxidil is 2.0%, the amount of carbomer is 0.5%, the amount of propylene glycol is 20%, the amount of ethanol is 40%, and the amount of DIPA is 0.5%.

5. A gel of claim 2, wherein the amount of minoxidil is 3%, the amount of carbomer is 0.5%, the amount of propylene glycol is 30%, the amount of ethanol is 40%, and the amount of DIPA is 0.5%.

6. A gel of any preceding claim, wherein the carbomer has a molecular weight of approx. $3 \times 10^6$.

7. A process for preparing a gel of any preceding claim, which comprises a first step of adding 10 to 27.1% w/w of the alcohol and 0 to 0.4% w/w of the amine to a mixture of the water and the carbomer; and a subsequent second step of adding to the mixture of the first step, until a uniform gel is obtained, a mixture of the minoxidil, the glycol, 12.9 to 30% w/w of the alcohol, and at least 0.25% w/w of the amine; the percentages being with respect to the total composition.

8. A process of claim 7, wherein no amine is present in the first step.

9. A process of claim 8, for preparing a gel of any of claims 3 to 5, wherein the carbomer is as defined in claim 6, and 27% w/w of the ethanol is added in the first step.

**Patentansprüche**

1. Pharmazeutisch akzeptable einphasige Minoxidilgelzubereitung, umfassend die folgenden Komponenten:

| Komponente | Menge (% G/G) |
|---|---|
| (a) Wasser | in genügender Menge 100 |
| (b) Carbomer | 0,25 - 1,5 |
| (c) Minoxidil | 0,001 - 3 |
| (d) pharmazeutisch akzeptables Glykol | 0,01 - 30 |
| (e) Ethanol oder Isopropanol | 20 - 40 |
| (f) ein wasser- und alkohollösliches Amin | 0,25 - 1,5 |

wobei das Verhältnis Minoxidil/Glykol für eine gesättigte Minoxidillösung ausreicht.

2. Gel nach Anspruch 1, wobei das Amin aus Diisopropanolamin (DIPA) und der Alkohol aus Ethanol bestehen, das Wasser gereinigt ist und das Glykol aus Propylenglykol besteht.

3. Gel nach Anspruch 2, wobei die Menge an Minoxidil 1,0%, an Carbomer 0,45%, an Propylenglykol 10%, an Ethanol 40% und an DIPA 0,45% beträgt.

4. Gel nach Anspruch 2, wobei die Menge an Minoxidil 2,0%, an Carbomer 0,5%, an Propylenglykol 20%, an Ethanol 40% und an DIPA 0,5% beträgt.

5. Gel nach Anspruch 2, wobei die Menge an Minoxidil 3%, an Carbomer 0,5%, an Propylenglykol 30%, an Ethanol 40% und an DIPA 0,5% beträgt.

6. Gel nach einem der vorhergehenden Ansprüche, wobei das Carbomer ein Molekulargewicht von etwa 3 x $10^6$ aufweist.

7. Verfahren zur Zubereitung eines Gels nach einem der vorhergehenden Ansprüche, bei welchem in einer ersten Stufe 10 - 27,1% G/G des Alkohols und 0 - 0,4% G/G des Amins zu einem Gemisch des Wassers und des Carbomers und in einer folgenden zweiten Stufe zu dem in der ersten Stufe erhaltenen Gemisch bis zum Erhalt eines gleichförmigen Gels ein Gemisch des Minoxidils, des Glykols, 12,9 - 30% G/G des Alkohols und mindestens 0,25% G/G des Amins (die Prozentangaben beziehen sich auf die gesamte Zubereitung) zugegeben werden.

8. Verfahren nach Anspruch 7, wobei in der ersten Stufe kein Amin vorhanden ist.

9. Verfahren nach Anspruch 8 zur Zubereitung eines Gels nach einem der Ansprüche 3 bis 5, wobei das Carbomer in Anspruch 6 definiert ist und in der ersten Stufe 27% G/G des Ethanols zugegeben werden.

**Revendications**

1. Composition de gel monophasique de minoxidil, pharmaceutiquement acceptable, comprenant les constituants suivants :

| Constituant | Quantité, (% en poids) |
|---|---|
| (a) eau | q.s. 100 |
| (b) carbomère | 0,25-1,5 |
| (c) minoxidil | 0,001-3 |
| (d) glycol pharmaceutiquement acceptable | 0,01-30 |
| (e) éthanol ou isopropanol | 20-40 |
| (f) une amine soluble dans l'eau et les alcools | 0,25-1,5 |

le rapport du minoxidil au glycol étant suffisant pour obtenir une solution de minoxidil saturée.

2.  Gel suivant la revendication 1, dans lequel l'amine est la diisopropanolamine (DIPA), l'alcool est l'éthanol, l'eau est de l'eau purifiée et le glycol est le propylèneglycol.

3.  Gel suivant la revendication 2, dans lequel la quantité de minoxidil est égale à 1,0 %, la quantité de carbomère est égale à 0,45 %, la quantité de propylèneglycol est égale à 10 %, la quantité d'éthanol est égale à 40 % et la quantité de DIPA est égale à 0,45 %.

4.  Gel suivant la revendication 2, dans lequel la quantité de minoxidil est égale à 2,0 %, la quantité de carbomère est égale à 0,5 %, la quantité de propylèneglycol est égale à 20 %, la quantité d'éthanol est égale à 40 % et la quantité de DIPA est égale à 0,5 %.

5.  Gel suivant la revendication 2, dans lequel la quantité de minoxidil est égale à 3 %, la quantité de carbomère est égale à 0,5 %, la quantité de propylèneglycol est égale à 30 %, la quantité d'éthanol est égale à 40 % et la quantité de DIPA est égale à 0,5 %.

6.  Gel suivant l'une quelconque des revendications précédentes, dans lequel le carbomère possède un poids moléculaire d'approximativement $3 \times 10^6$.

7.  Procédé de préparation d'un gel suivant l'une quelconque des revendications précédentes, qui comprend une première étape d'addition de 10 à 27,1 % en poids/poids de l'alcool et de 0 à 0,4 % en poids/poids de l'amine à un mélange de l'eau et du carbomère ; puis une seconde étape d'addition au mélange de la première étape, jusqu'à obtention d'un gel uniforme, d'un mélange du minoxidil, du glycol, de 12,9 à 30 % en poids/poids de l'alcool et d'au moins 0,25 % en poids/poids de l'amine, les pourcentages étant exprimés sur la base de la composition totale.

8.  Procédé suivant la revendication 7, dans lequel aucune amine n'est présente dans la première étape.

9.  Procédé suivant la revendication 8, pour la préparation d'un gel suivant l'une quelconque des revendications 3 à 5, dans lequel le carbomère répond à la définition suivant la revendication 6 et 27 % en poids/poids de l'éthanol sont ajoutés dans la première étape.